# EUROPÄISCHE PATENTSCHRIFT

(11) **EP 1 647 824 B1**
(45) Veröffentlichungstag und Bekanntmachung des Hinweises auf die Patenterteilung: **22.12.2010**
(21) Anmeldenummer: 05022253.8
(22) Anmeldetag: 12.10.2005
(51) Int. Cl.: G01N 33/487

(54) **Vorrichtung, Messgerät und Verfahren zur Handhabung mikrofluidischer Plattformen**
Device, measuring apparatus and method for handling microfluidic platforms
Dispositif, appareil de mesure, et procédé pour la manipulation de plateformes microfluidiques

(30) Priorität: 13.10.2004 DE 102004050062
(43) Veröffentlichungstag der Anmeldung: 19.04.2006
(60) Teilanmeldung: 07023722.7
(73) Patentinhaber: Boehringer Ingelheim microParts GmbH, 44227 Dortmund (DE)
(72) Erfinder: Dechant, Christian, 44795 Bochum (DE); Kadel, Klaus, Dr., 58453 Witten (DE); Blankenstein, Gert, Dr., 44139 Dortmund (DE)
(74) Vertreter: Gesthuysen, von Rohr & Eggert

(56) Entgegenhaltungen:
- DE-A1- 4 326 339
- DE-A1- 10 244 775
- DE-A1- 19 815 684
- US-A- 5 514 152
- US-A1- 2003 024 811

## Beschreibung

Die vorliegende Erfindung betrifft eine Vorrichtung mit einer Vielzahl von mikrofluidischen Plattformen zur Aufnahme und insbesondere Untersuchung oder Manipulation von Probenflüssigkeit gemäß dem Oberbegriff des Anspruchs 1 sowie ein Verfahren zur Untersuchung oder Manipulation von Probenflüssigkeit gemäß dem Oberbegriff des Anspruchs 7.

Der Begriff "mikrofluidische Plattform" - nachfolgend oft kurz als Plattform bezeichnet - umfaßt bei der vorliegenden Erfindung Probenträger, Teststreifen oder dgl., die insbesondere durch Kapillarkräfte eine zu untersuchende oder zu manipulierende Probenflüssigkeit aufnehmen können. Insbesondere dienen die Plattformen zur Durchführung einzelner Tests oder Messungen, beispielsweise der Glucose-Bestimmung von Blut. Jedoch können die Plattformen auch jeder sonstigen Untersuchung, insbesondere von Körperflüssigkeiten von Menschen oder Tieren als Probenflüssigkeit dienen. Zur Untersuchung bzw. Manipulation von Probenflüssigkeit weisen die Plattformen insbesondere Reagenzien, Filter oder dgl. für die Probenflüssigkeit auf. Zur Bevorratung bzw. Lagerung sind die Plattformen üblicherweise verschlossen.

Derartige Plattformen werden beispielsweise in Form von Teststreifen zur Bestimmung des Blutzuckerspiegels (Glucose-Bestimmung) verwendet. Bisher ist es hierbei erforderlich, einen einzelnen Teststreifen aus einer Vorratspackung zu entnehmen und mit Blut als Probenflüssigkeit zu benetzen, die dann von dem Teststreifen aufgenommen wird. Anschließend muß der Teststreifen manuell in ein entsprechendes Meß- bzw. Testgerät eingesetzt werden, das dann zunächst anzeigt, ob in ausreichender Menge Blut aufgenommen wurde und schließlich den Glucosewert bestimmt. Anschließend muß der Teststreifen wieder entnommen und entsorgt werden. Der beschriebene Ablauf ist aufwendig und mit gewissen Unsicherheiten verbunden. Die Handhabung des verhältnismäßig kleinen Teststreifens ist insbesondere für ungeübte Benutzer schwierig. Es besteht das Risiko einer unerwünschten Kontaminierung. Des weiteren wird oftmals Blut in nicht ausreichender Menge aufgenommen, was jedoch erst nach dem Einsetzen des Teststreifens in das Meßgerät feststellbar ist, so daß dann die gleiche Prozedur noch einmal durchlaufen werden muß.

Aus der WO 03/045557 A2, die den Ausgangspunkt der vorliegenden Erfindung bildet, ist eine bandartige Anordnung von einer Vielzahl von Plattformen bekannt, die hintereinander für eine Vielzahl von unmittelbar aufeinander folgenden, automatisierten Untersuchungen bzw. Manipulation von Probenflüssigkeiten einsetzbar sind. Die bekannte Anordnung ist jedoch nicht zur einzelweisen Untersuchung bzw. Manipulation von Probenflüssigkeit vorgesehen oder geeignet.

Die US 5,514,152 A betrifft eine Vorrichtung mit einer Vielzahl mikrofluidischer Plattformen zur Aufnahme und Untersuchung von Probeflüssigkeit. Die Plattformen sind im verschlossenen Zustand miteinander verbunden und können jeweils einzelweise abgetrennt und geöffnet werden. Weiterhin wird ein Gerät und ein Verfahren zur Verwendung der Vorrichtung offenbart.

Der vorliegenden Erfindung liegt die Aufgabe zugrunde, eine Vorrichtung mit einer Vielzahl von mikrofluidischen Plattformen zur Aufnahme und insbesondere Untersuchung oder Manipulation von Probenflüssigkeiten, ein Meßgerät zur Untersuchung von Probenflüssigkeit mittels einer mikrofluidischen Plattform und ein Verfahren zur Untersuchung oder Manipulation von Probenflüssigkeit mittels einer mikrofluidischen Plattform anzugeben, wobei eine einfache bedarfsgerechte einzelweise Aufnahme und insbesondere Untersuchung oder Manipulation von Probenflüssigkeit, vorzugsweise also einzelne Untersuchungen bzw. Manipulationen unabhängig von vorherigen Untersuchungen bzw. Manipulationen, ermöglicht werden, wobei insbesondere aus hygienischen Gründen jede benutzte Plattform unmittelbar nach ihrer Benutzung und unabhängig von den anderen Plattformen entsorgt werden kann.

Die obige Aufgabe wird durch eine Vorrichtung gemäß Anspruch 1 oder ein Verfahren gemäß Anspruch 7 gelöst. Vorteilhafte Weiterbildungen sind Gegenstand der Unteransprüche.

Ein Aspekt der vorliegenden Erfindung liegt darin, bei einer Vielzahl von Plattformen, die insbesondere in einer bandförmigen, beispielsweise aufgewickelten oder gestapelten Anordnung vorliegen, die Plattformen einzelweise zu separieren und einzelweise zu öffnen, so daß bedarfsgerecht die Plattformen einzelweise zur Aufnahme und insbesondere Untersuchung oder Manipulation von Probenflüssigkeit eingesetzt und anschließend - unabhängig von den noch nicht benutzten Plattformen - entsorgt werden können. Dies ermöglicht eine wesentlich vereinfachte Handhabung für einen Benutzer, der beispielsweise in gewissen Zeitabständen seinen Blutzuckerspiegel überprüfen muß. Erfindungsgemäß ist es dann nämlich möglich, ein die Vielzahl von Plattformen enthaltendes Meßgerät einzusetzen, das die Bestimmung des Blutzuckerwerts (Glucose-Bestimmung) bedarfsgerecht gestattet, ohne daß der Benutzer zunächst einen einzelnen Teststreifen nach der Aufnahme von Blut in das Meßgerät einführen und anschließend wieder entfernen muß. Vielmehr ist das Meßgerät nach einem vorzugsweise automatisierten Öffnen einer Plattform direkt zur Aufnahme von Blut und beispielsweise Bestimmung des Glucosewerts einsetzbar, wobei unmittelbar nach der Bestimmung die benutzte Plattform vorzugsweise direkt ausgegeben und entsorgt werden kann. Dies ermöglicht eine optimale bedarfsgerechte und hygienische Benutzung bei einfacher Handhabung.

Besonders bevorzugt werden die einzelnen Plattformen jeweils zwangsweise durch das Separieren zur Aufnahme von Probenflüssigkeit geöffnet. Dies gestattet eine sehr einfache Handhabung und Benutzung der einzelnen Plattformen.

Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnung. Es zeigt:
- Fig. 1: eine ausschnittsweise Draufsicht einer vorschlagsgemäßen Vorrich- tung mit einer Vielzahl von Plattformen gemäß einer ersten Ausfüh- rungsform;
- Fig. 2: eine schematische Darstellung einer separierten Plattform der Vor- richtung gemäß der ersten Ausführungsform bei der Aufnahme von Probenflüssigkeit; und
- Fig. 3: einen Schnitt entlang Linie III - IV von Fig. 1.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

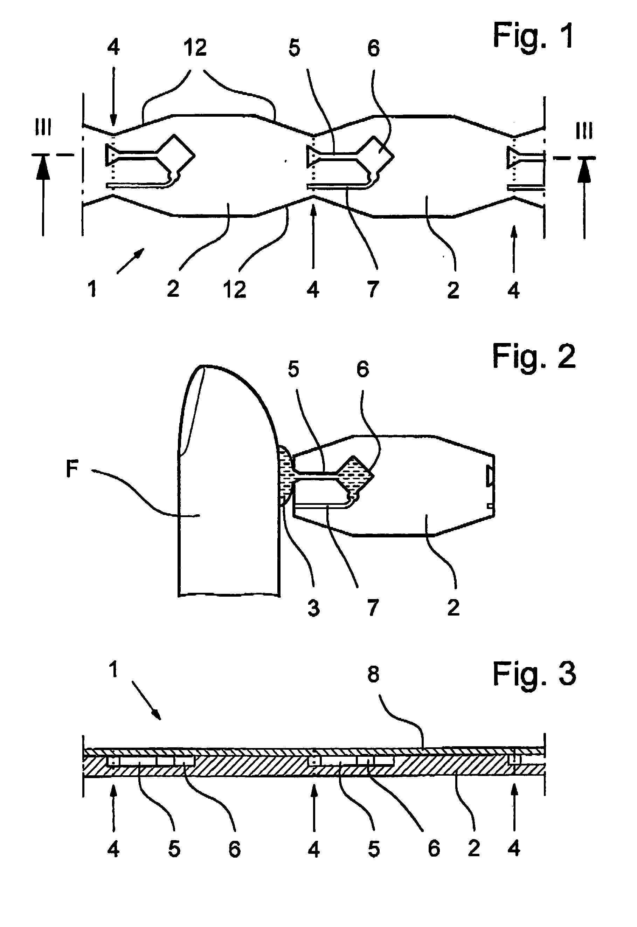

Fig. 1 zeigt eine vorschlagsgemäße Vorrichtung 1 gemäß einer ersten Ausführungsform mit einer Vielzahl von mikrofluidischen Plattformen 2 im eingangs genannten Sinne. Die Plattformen 2 dienen insbesondere der Aufnahme und Untersuchung oder Manipulation von Probenflüssigkeit 3, beispielsweise Blut zur Glucose-Bestimmung, wie in Fig. 2 beispielhaft für eine separierte Plattform 2 dargestellt, die die Probenflüssigkeit 3 von einem Finger F bereits aufgenommen hat.

Bei der in Fig. 1 bis 3 dargestellten ersten Ausführungsform der Vorrichtung 1 sind die Plattformen 2 im ungebrauchten und verschlossenen Zustand unmittelbar miteinander verbunden, und zwar über gestrichelt angedeutete Verbindungen 4. Diese Verbindungen 4 sind vorzugsweise als Sollbruchstellen ausgebildet.

Jede Plattform 2 weist vorzugsweise einen Aufnahmekanal 5 zur Aufnahme von Probenflüssigkeit 3, eine sich anschließende Probenkammer 6, die insbesondere einer Untersuchung und/oder Manipulation der Probenflüssigkeit 3 - bedarfsweise unter Einwirkung von einem vorher in die Probenkammer 6 eingebrachten, nicht dargestellten, insbesondere eingetrockneten Reagenz oder dgl. - dient, und bedarfsweise einen sich an die Probenkammer 6 anschließenden Entlüftungskanal 7 auf. Vorzugsweise ist am Ausgang der Probenkammer 6 zum Entlüftungskanal 7 hin ein Flüssigkeitsstop gebildet, so daß die vorzugsweise nur durch Kapillarkräfte in den Aufnahmekanal 5 und die Probenkammer 6 einströmende Probenflüssigkeit 3 nicht in den Entlüftungskanal 7 gesaugt wird bzw. eintritt, wie in Fig. 2 angedeutet.

Dem Schnitt gemäß Fig. 3 ist zu entnehmen, daß die Kavitäten der Plattformen 2, wie der Aufnahmekanal 5, die Probenkammer 6 und der Entlüftungskanal 7, vorzugsweise in einem geeigneten Material, vorzugsweise Kunststoff oder ggf. Glas, bei der Darstellung nach oben offen ausgebildet und durch eine Abdeckung 8 abgedeckt, also oberseitig geschlossen sind. Die Abdeckung 8 ist beim Darstellungsbeispiel vorzugsweise in Richtung des bandförmigen Verlaufs durchgehend ausgebildet und ggf. auch mit Sollbruchstellen entsprechend den Verbindungen 4 versehen.

Die Abdeckung 8 besteht vorzugsweise aus Folie. Sie kann jedoch aus jedem sonstigen geeigneten Material gebildet sein. Vorzugsweise ist die Abdeckung 8 aufkaschiert, verschweißt, aufgeklebt oder in sonstiger geeigneter Weise mit dem die Kavitäten der Plattformen 2 bildenden, plattenförmigen Material verbunden, um das vorzugsweise gewünschte hermetische Schließen der Kavitäten zu gewährleisten.

Bei der ersten Ausführungsform erstrecken sich der Aufnahmekanal 5 und der Entlüftungskanal 7 über die Verbindung 4 hinweg jeweils bis in die nachfolgende Plattform 2. Auf diese Weise sind bei den zusammenhängenden Plattformen 2 die Aufuahmekanäle 5 und die Entlüftungskanäle 7 geschlossen, die einzelnen Plattformen 2 also zur Aufnahme von Probenflüssigkeit 3 noch nicht geöffnet.

Die vorgenannte Ausbildung führt dazu, daß die Plattformen 2 einzelweise separierbar und einzelweise öffenbar sind. Insbesondere werden bei der ersten Ausführungsform die einzelnen Plattformen 2 jeweils zwangsweise durch das Separieren - also Trennen im Bereich der Verbindung 4 - zur Aufnahme von Probenflüssigkeit 3 geöffnet, da hierdurch jeweils der entsprechende Aufnahmekanal 5 und Entlüftungskanal 7 geöffnet werden. Anschließend ist jede Plattform 2 einzelweise zur Aufnahme von Probenflüssigkeit 3, wie in Fig. 2 gezeigt, und für eine anschließende Untersuchung bzw. Manipulation der Probenflüssigkeit 3 in der Probenkammer 6 bereit.

Die Plattformen 2 können je nach Bedarf und Ausbildung beispielsweise durch Schneiden, Brechen oder Reißen im Bereich ihrer Verbindungen 4 - wahlweise manuell oder insbesondere mittels einer geeigneten Einrichtung - bedarfsgerecht und einfach voneinander getrennt werden, wobei die vorzugsweise vorgesehenen Sollbruchstellen jedoch nicht unbedingt erforderlich sind.

Die Vorrichtung 1 gemäß der ersten Ausführungsform ist vorzugsweise derart ausgebildet, daß sie bzw. die noch nicht separierten Plattformen 2 aufwickelbar oder stapelbar sind, insbesondere aufgrund eigener Flexibilität der Plattformen 2 und/oder des Trägers 10 und/oder aufgrund entsprechend flexibler Verbindungen 4.

Die Vorrichtung 1 bzw. die Plattformen 2 ist bzw. sind vorzugsweise mit mechanischen und/oder optischen Markierungen, Aussparungen, Löchern, Schwächungen Verjüngungen 12 (Fig. 1) oder Hinterschneidungen versehen, um das Fördern, Positionieren, Erfassen und/oder Separieren einzelner Plattformen 2 zu ermöglichen oder zu erleichtern.

Es ist anzumerken, daß je nach Konstruktion und Aufbau der Plattformen 2 ggf. auch ganz auf eine nachträglich aufgebrachte Abdeckung 8 verzichtet werden kann. Jedoch gestattet die Ausbildung von zu einer Flachseite hin offenen Kavitäten, eine sehr einfache Herstellung und insbesondere bedarfsweise auch eine vorherige Einbringung von Reagenzien in die Probenkammer 6; die Abdeckung 8 ist dann zum Verschließen der Plattformen 2 erforderlich.

## Patentansprüche

1. Vorrichtung (1) mit einer Vielzahl von mikrofluidischen Plattformen (2) zur Aufnahme und insbesondere Untersuchung oder Manipulation von Probenflüssigkeit (3), wie Blut zur Glucose-Bestimmung, wobei die Plattformen (2) im verschlossenen Zustand unmittelbar miteinander verbunden, von einer vorzugsweise durchgehenden Abdeckung (8) abgedeckt und/oder auf einem gemeinsamen Träger (10) angeordnet sind, wobei die Plattformen (2) jeweils einzelweise separierbar und einzelweise öffenbar sind und bei den unmittelbar miteinander über Verbindungen (4) verbundenen Plattformen (2) jede Plattform (2) einen Aufnahmekanal (5) zur Aufnahme von Probenflüssigkeit (3) aufweist, der sich bis zur oder über die Verbindung (4) bis in die nachfolgende Plattform (2) erstreckt bzw. erstrecken, so daß der Aufnahmekanal (5) mit dem Trennen der Verbindung (4) geöffnet wird,
**dadurch gekennzeichnet,**
**daß** jede Plattform (2) einen Entlüftungskanal (7) aufweist, der sich bis zur oder über die Verbindung (4) bis in die nachfolgende Plattform (2) erstreckt, so daß der Entlüftungskanal (7) mit dem Trennen der Verbindung (4) geöffnet wird, und
**daß** zwangsweise durch das Separieren einer Plattform (2) nur diese zur Aufnahme von Probenflüssigkeit (3) geöffnet wird.

2. Vorrichtung nach Anspruch 1, **dadurch gekennzeichnet, daß** die Abdeckung (8) mindestens eine Kavität, vorzugsweise alle Kavitäten, wie den Aufnahmekanal (5), eine Probenkammer (6) und/oder den Entlüftungskanal (7) bzw. deren Öffnungen der Plattformen (2), abdeckt und/oder daß die Abdeckung (8) aus Folie besteht.

3. Vorrichtung nach einem der voranstehenden Ansprüche, **dadurch gekennzeichnet, daß** die Plattformen (2) bandförmig hintereinander angeordnet und/oder jeweils gleichzeitig separierbar und öffenbar sind.

4. Vorrichtung nach einem der voranstehenden Ansprüche, **dadurch gekennzeichnet, daß** die Verbindungen (4) der unmittelbar miteinander verbundenen Plattformen (2) als Sollbruchstellen ausgebildet sind, und/oder daß der Träger (10) bandförmig ausgebildet ist und/oder aus Folie besteht.

5. Vorrichtung nach einem der voranstehenden Ansprüche, **dadurch gekennzeichnet daß** die noch nicht separierten Plattformen (2) aufwickelbar sind, insbesondere aufgrund eigener Flexibilität, aufgrund einer flexiblen gegenseitigen Verbindung (4) und/oder aufgrund einer Flexibilität des Trägers (10), und/oder daß die noch nicht separierten Plattformen (2) stapelbar sind, insbesondere aufgrund einer flexiblen gegenseitigen Verbindung (4) und/oder aufgrund einer Flexibilität des Trägers (10).

6. Vorrichtung nach einem der voranstehenden Ansprüche, **dadurch gekennzeichnet, daß** die Vorrichtung (1), insbesondere die Plattformen (2), der Träger (10) und/oder eine Abdeckung (8) der Plattformen (2), mit mechanischen und/oder optischen Markierungen, Aussparungen, Löchern, Verjüngungen (12), Schwächungen oder Hinterscheidungen versehen sind, um das Fördern, Positionieren, Erfassen und/oder Separieren einzelner Plattformen (2) zu ermöglichen.

7. Verfahren zur Untersuchung oder Manipulation von Probenflüssigkeit (3), wie Blut zur Glucose-Bestimmung, mittels einer mikrofluidischen, die Probenflüssigkeit (3) aufnehmenden Plattform (2), insbesondere mittels einer Vorrichtung (1) gemäß einem der Ansprüche 1 bis 9, wobei eine Vielzahl von Plattformen (2) zugefördert werden, die im verschlossenen Zustand unmittelbar miteinander verbunden, von einer vorzugsweise gemeinsamen Abdeckung (8) abgedeckt und/oder auf einem gemeinsamen Träger (10) angeordnet sind, wobei die Plattformen (2) einzelweise und nur bedarfsweise zur unmittelbar anschließenden Untersuchung bzw. Manipulation von Probenflüssigkeit (3) geöffnet und separiert werden, wobei die Plattformen (2) einzelweise durch Trennen einer Verbindung (4) zu einer benachbarten Plattform (2) geöffnet werden,
**dadurch gekennzeichnet,**
**daß** durch das Trennen der Verbindung (4) ein Entlüftungskanal (7) zusammen mit einem Aufnahmekanal (5) der Plattform (2) geöffnet wird, und
**daß** nur die abgetrennte Plattform (2) zur Aufnahme von Probenflüssigkeit (3) geöffnet wird.

## Claims

1. Apparatus (1) having a plurality of microfluidic platforms (2) for receiving and in particular investigating or manipulating sample liquid (3), such as blood for measuring glucose levels, wherein the platforms (2) are directly connected to one another in the closed state, covered by a preferably continuous cover (8) and/or arranged on a common carrier (10), wherein the platforms (2) can each be individually detached and individually opened and, in the platforms (2) that are directly connected to one another by connections (4), each platform (2) has a receiving channel (5) for receiving sample liquid (3), which extend or extends up to or through the connection (4) into the next platform (2), so that the receiving channel (5) is opened when the connection (4) is severed,
**characterised in that**
each platform (2) has a venting channel (7) which extends up to or through the connection (4) into the next platform (2), so that the venting channel (7) is opened when the connection (4) is severed, and
as a result of the detachment of a platform (2) only this platform is forcibly opened to receive sample liquid (3).

2. Apparatus according to claim 1, **characterised in that** the cover (8) covers at least one cavity, preferably all the cavities, such as the receiving channel (5), a sample chamber (6) and/or the venting channel (7) or the openings thereof in the platforms (2), and/or the cover (8) consists of film.

3. Apparatus according to one of the preceding claims, **characterised in that** the platforms (2) are arranged in a strip one behind the other and/or can be detached and opened simultaneously in each case.

4. Apparatus according to one of the preceding claims, **characterised in that** the connections (4) between the platforms (2) that are directly connected to one another are constructed as frangible points, and/or the carrier (10) is constructed in the form of a strip and/or consists of film.

5. Apparatus according to one of the preceding claims, **characterised in that** the platforms (2) that are not yet detached can be wound in a coil, particularly by virtue of their inherent flexibility, a flexible mutual connection (4) and/or a flexibility of the carrier (10), and/or the platforms (2) that are not yet detached can be stacked, particularly by virtue of a flexible mutual connection (4) and/or a flexibility of the carrier (10).

6. Apparatus according to one of the preceding claims, **characterised in that** the apparatus (1), particularly the platforms (2), the carrier (10) and/or a cover (8) of the platforms (2), are provided with mechanical and/or optical markings, cut-outs, holes, tapers (12), weakened points or undercuts, to allow the conveying, positioning, gripping and/or detachment of individual platforms (2).

7. Process for investigating or manipulating sample liquid (3) such as blood for measuring glucose levels, by means of a microfluidic platform (2) that receives the sample liquid (3), in particular by means of an apparatus according to one of claims 1 to 6, wherein a plurality of platforms (2) are delivered which are directly connected to one another in the closed state, are covered by a preferably common cover (8) and/or arranged on a common carrier (10), wherein the platforms (2) can each be opened and detached individually and only as required for the immediate investigation or manipulation of sample liquid (3), the platforms (2) being opened individually by the severing of a connection (4) to an adjacent platform (2), **characterised in that**
as a result of the severing of the connection (4) a venting channel (7) together with a receiving channel (5) of the platform (2) is opened up, and
only the platform (2) that has been detached is opened up to receive sample liquid (3).

## Revendications

1. Dispositif (1) avec une pluralité de plateformes microfluidiques (2) pour la réception et en particulier l'examen ou la manipulation de liquide de prélèvement (3) tel que le sang pour la détermination du glucose, les plateformes (2) étant reliées les unes aux autres directement à l'état fermé, recouvertes par un recouvrement (8) de préférence traversant et/ou sont disposées sur un support commun (10), les plateformes (2) pouvant être séparées respectivement individuellement et ouvertes individuellement et pour les plateformes (2) reliées directement les unes aux autres par des liaisons (4), chaque plateforme (2) présentant un canal de réception (5) pour la réception de liquide de prélèvement (3) qui s'étend jusqu'à ou par le biais de la liaison (4) jusque dans la plateforme suivante (2) de sorte que le canal de réception (5) soit ouvert en même temps que la liaison (4) est séparée,
**caractérisé en ce**
**que** chaque plateforme (2) présente un canal d'aération (7) qui s'étend jusqu'à ou par le biais de la liaison (4) jusque dans la plateforme suivante (2) de sorte que le canal d'aération (7) soit ouvert en même temps que la liaison (4) est séparée et
en ce que forcément par la séparation d'une plateforme (2), seule celle-ci est ouverte pour la réception de liquide de prélèvement (3).

2. Dispositif selon la revendication 1, **caractérisé en ce que** le recouvrement (8) recouvre au moins une cavité, de préférence toutes les cavités comme le canal de réception (5), une chambre de prélèvement (6) et/ou le canal d'aération ou leurs ouvertures des plateformes (2) et/ou **en ce que** le recouvrement (8) se compose de film.

3. Dispositif selon l'une quelconque des revendications précédentes, **caractérisé en ce que** les plateformes (2) sont disposées en forme de bande les unes derrière les autres et/ou peuvent être séparées et ouvertes respectivement en même temps.

4. Dispositif selon l'une quelconque des revendications précédentes, **caractérisé en ce que** les liaisons (4) des plateformes (2) reliées directement les unes aux autres sont réalisées comme des points destinés à la rupture et/ou **en ce que** le support (10) est réalisé en forme de bande et/ou se compose de film.

5. Dispositif selon l'une quelconque des revendications précédentes, **caractérisé en ce que** les plateformes qui ne sont pas encore séparées (2) peuvent être enroulées, en particulier en raison de leur propre flexibilité, en raison d'une liaison mutuelle flexible (4) et/ou en raison d'une flexibilité du support (10) et/ou **en ce que** les plateformes qui ne sont pas encore séparées (2) peuvent être empilées, en particulier en raison d'une liaison mutuelle flexible (4) et/ou en raison d'une flexibilité du support (10).

6. Dispositif selon l'une quelconque des revendications précédentes, **caractérisé en ce que** le dispositif (1), en particulier les plateformes (2), le support (10) et/ou un recouvrement (8) des plateformes (2) sont pourvus de marquages mécaniques, et/ou optiques, d'évidements, de trous, de rétrécissements (12), d'affaiblissements ou de contre-dépouilles afin de permettre le transport, le positionnement, la saisie et/ou la séparation de plateformes individuelles (2).

7. Procédé d'examen ou de manipulation de liquide de prélèvement (3) tel que du sang pour la détermination du glucose, à l'aide d'une plateforme (2) microfluidique, recevant le liquide de prélèvement (3), en particulier à l'aide d'un dispositif selon l'une quelconque des revendications 1 à 9, une pluralité de plateformes (2) étant transportée, lesquelles sont reliées directement les unes aux autres à l'état fermé, recouvertes par un recouvrement (8) de préférence commun et/ou sont disposées sur un support commun (10), les plateformes (2) étant ouvertes et séparées individuellement et seulement si besoin est, pour l'examen ou la manipulation directement consécutive de liquide de prélèvement (3), les plateformes (2) étant ouvertes individuellement par la séparation d'une liaison (4) avec une plateforme (2) contigüe,
**caractérisé en ce**
**que** par la séparation de la liaison (4) est ouvert un canal d'aération (7) conjointement avec un canal de réception (5) de la plateforme (2) et
en ce que seule la plateforme séparée (2) est ouverte pour la réception de liquide de prélèvement (3).
